**Europäisches Patentamt**

**European Patent Office**

**Office européen des brevets**

(19)

(11) Publication number : **0 321 523 B1**

# (12) EUROPEAN PATENT SPECIFICATION

(45) Date of publication of patent specification :
02.12.92 Bulletin 92/49

(51) Int. Cl.⁵ : **G01N 21/75, G01N 33/543**

(21) Application number : **88905401.1**

(22) Date of filing : **17.06.88**

(86) International application number :
**PCT/GB88/00471**

(87) International publication number :
**WO 88/10418 29.12.88 Gazette 88/28**

(54) **WAVEGUIDE SENSOR.**

(30) Priority : **20.06.87 GB 8714503**

(43) Date of publication of application :
**28.06.89 Bulletin 89/26**

(45) Publication of the grant of the patent :
**02.12.92 Bulletin 92/49**

(84) Designated Contracting States :
**AT BE CH DE FR GB IT LI LU NL SE**

(56) References cited :
**WO-A-84/02578**
**GB-A- 2 174 802**
**OPTICS LETTERS vol. 9, no. 4, April 1984,**
**Optical Society of America, (New York, US);**
**K.TIEFENTHALER et al.: "Integratedoptical**
**switches and gas sensors", pages 137-139**

(56) References cited :
**CLINICAL CHEMISTRY, vol. 30, no. 9, September 1984, (Winston, US); R.M.SUTHERLAND et al.: "Optical detection ofantibody-antigen reactions at a glass-liquid interface", pages 1533-1538**

(73) Proprietor : **APPLIED RESEARCH SYSTEMS ARS HOLDING N.V.**
**6 John B. Gorsiraweg**
**Curacao (AN)**

(72) Inventor : **SAWYERS, Craig, George**
**The Laurels 11 Howitts Lane**
**Eynesbury, St. Neots Cambridgeshire (GB)**

(74) Representative : **Woodman, Derek et al**
**FRANK B. DEHN & CO. Imperial House 15-19 Kingsway**
**London WC2B 6UZ (GB)**

## Description

Field of the invention

This invention concerns assay techniques by which qualitative and/or quantitative detection of chemical, biochemical or biological species in a sample can be effected and also concerns apparatus suitable for use in such techniques.

Background to the invention

The assay techniques with which the present application is concerned are based on the affinity between the species which is to be assayed (hereinafter called "ligand") and a specific binding material for the ligand (hereinafter called "specific binding partner") which is coated on a particular type of surface. K. Tiefenthaler and W. Lukosz, Optics Letters 10, 137 (1984) disclose directional-switching effects in planar optical waveguides with surface gratings. Switching is actuated by adsorption or desorption of water or other adsorbates on the waveguide surface through a change in the effective index of the guided modes under the grating, said guided modes being excited by means of a monochromatic light source, namely a laser beam. Published application WO86/07149 describes the application of this technique to the selective detection of specific substances in gaseous, liquid, solid or porous samples. International Patent Publications WO84/02578 and WO86/01901 describe assay techniques comprising (a) coating at least a part of a surface (being of preformed relief profile) on a substrate with a thin film of a specific binding partner, the said part of the surface being optically active with respect to radiation over a band of wavelengths, and (b) contacting the coated surface with a sample and observing the optical properties of the said part of the surface in order to determine a qualitative and/or quantitative change in optical properties as a result of the binding of the ligand onto the thin film of specific binding partner on the surface.

As described in WO84/02578 and WO86/01901, the preformed relief profile is typically in the form of an optical grating which may be a simple single grating or two or more crossed gratings, the ridges of which may, for example, have square, sinusoidal or triangular cross-sectional shape, and as employed herein references to a grating are intended to encompass all such gratings.

In publication WO84/02578, the change in optical properties of the grating as a result of the binding of the ligand to be assayed (such as a specific antigen in blood serum) is brought about essentially as a result of (1) the mass or bulk of the bound ligand and (2) its dielectric properties.

In publication WO86/01901 the binding events are monitored by changes in the fluorescent properties of a dye-tagged binding partner attached to the sensor surface.

It is an object of the present invention to provide an alternative method of assay involving a more sensitive type of sensor.

Summary of the invention

Thus, in its broadest aspect, the invention provides a method of assaying for a ligand in a sample which comprises

a) incubating the sample in contact with the surface of an optical structure, said optical structure comprising a substrate with a first refractive index N1 and a surface layer with a second refractive index N2, said surface layer being such that, in use, a guided wave mode of resonant radiation may be propagated therein, and said surface layer having adsorbed thereon or bound thereto, either directly or indirectly, a specific binding partner for the ligand it is desired to detect;

b) irradiating the optical structure with polychromatic light (i.e. light which is neither monochromatic nor quasi-monochromatic) at a fixed angle to the normal; and

c) analysing the reflected and/or transmitted light in order to determine whether, and if desired the extent to which and/or rate at which, the absorption of any wavelengths of light as a result of guided wave resonance is altered by formation of a complex between the ligand and the specific binding partner.

It will be appreciated that, in order to propagate a guided wave mode within the surface layer, N2 must be greater than N1. However, it is particularly preferred that the refractive indices N1 and N2 are similar (but not precisely equal) and that both are approximately 1.5.

Preferably the optical structure is a diffraction grating with a substrate of clear plastics, for example polycarbonate, or glass and a surface layer of any suitable refractive index. It is advantageous to utilise an optical structure in the method according to the invention wherein the surface layer is less than 700 nm thick.

The surface layer of refractive index N2 may comprise a thin layer of material. For example, where the substrate of the optical structure is of plastics the thin layer of material thereon may be of glass or silica and where the substrate is of glass, for example phosphate glass, the thin layer of material may be of silica or spun-coated polymer, for example polystyrene. However, it is particularly preferred for the substrate of the optical structure to be of glass and for the surface layer of refractive index N2 to comprise the surface of said substrate impregnated with metal ions, for example silver and/or lead ions. One method of forming a surface layer of refractive index N2 on an optical structure as defined herein comprises exposing the surface of the optical structure to silver nitrate and/or lead nitrate.

A sensor for use in accordance with the invention comprises:

an optical structure having a substrate with a first refractive index N1 and a surface layer with a second refractive index N2, said surface layer being such that, in use, a guided wave mode of resonant radiation may be propagated therein, and said surface layer having adsorbed thereon or bound thereto, either directly or indirectly, a specific binding partner for the ligand it is desired to detect.

The invention is especially useful for the detection of antibodies or antigens [in which case the specific binding partner may be an antigen or an antibody (monoclonal or polyclonal) respectively] but other ligands may be detected by the use of other specific binding partners, as discussed hereinafter. However, the invention is not limited to the use of biosensors and may involve chemical sensors such as, for example, gaseous chemical detectors wherein specific adsorption onto or absorption into the surface layer may occur.

The invention will be particularly described hereinafter with reference to an antibody or an antigen as the specific binding partner or ligand. However, the invention is not to be taken as being limited to methods of antibody or antigen assay but includes within its scope assays which can be used to detect any chemical, biochemical or biological species in a sample. Examples of suitable binding partners which may be immobilised on an optical structure according to the invention and of ligands which may be assayed by the method of the invention are given in Table I below.

## Table I

| Ligand | Specific Binding Partner |
|---|---|
| antigen | specific antibody |
| antibody | antigen |
| hormone | hormone receptor |
| hormone receptor | hormone |
| polynucleotide strand | complementary polynucleotide strand |
| avidin | biotin |
| biotin | avidin |
| protein A | immunoglobulin |
| immunoglobulin | protein A |
| enzyme | enzyme cofactor (substrate) or inhibitor |
| enzyme cofactor (substrate) or inhibitor | enzyme |
| lectins | specific carbohydrate |
| specific carbohydrate of lectins | lectins |

The method of the invention has very broad applicability but in particular may be used to assay: hormones, including peptide hormones (e.g. thyroid stimulating hormone (TSH), luteinising hormone (LH), human chorionic gonadotronhin (hCG), follicle stimulating hormone (FSH), insulin and prolactin) and non-peptide hormones (e.g. steroid hormones such as cortisol, estradiol, progesterone and testosterone, or thyroid hormones such as thyroxine (T4) and triiodothyronine), proteins (e.g. carcino-embryonic antigen (CEA) and alphafetoprotein (AFP)), drugs (e.g. digoxin), sugars, toxins, vitamins, viruses, bacteria or microorganisms.

It will be understood that the term "antibody" used herein includes within its scope

a) any of the various classes or sub-classes of immunoglobulin, e.q. IgG, IgA, IgM or IgE, derived from any of the animals conventionally used, e.g. sheep, rabbits, goats or mice, or mice,

b) monoclonal antibodies,

c) intact molecules or "fragments" of antibodies, monoclonal or polyclonal, the fragments being those which contain the binding region of the antibody, i.e. fragments devoid of the Fc portion (e.g., Fab, Fab', F(ab')$_2$) or the so-called "half-molecule" fragments obtained by reductive cleavage of the disulphide bonds connecting the heavy chain comnonents in the intact antibody.

The method of preparation of fragments of antibodies is well known in the art and will not be described herein.

The term "antigen" as used herein will be understood to include both permanently antigenic species (for example, proteins, bacteria, bacteria fragments, cells, cell fragments and viruses) and haptens which may be rendered antigenic under suitable conditions.

The invention will be described in more detail with reference to a preferred embodiment wherein the optical structure is a diffraction grating. However, it is to be understood that other optical structures such as, for example, optical waveguides, optical fibres and coated prisms may, in the correct configuration, be included within the scope of the invention.

The invention relies for its effect, when observing the zero order diffraction efficiency of the system, on the sharp resonance anomaly due to excitation of guided waves in the triple layer dielectric arrangement formed between the optical structure as described herein, such as a coated diffraction grating, and the sample in contact with the surface layer of said optical structure. Whilst theoretically this resonance would not be expected to be observed, in practice there will usually be a sufficient attenuation of the incident radiation to observe the phenomenon. However, in some circumstances it may be desirable to encourage the absorption of coupled radiation by suitable alteration of one or more layers of the arrangement. Such an arrangement may consist of a substrate with a refractive index N1, coated with a conformal coating of refractive index N2 in contact with air or a liquid of a still further refractive index N3. By appropriate choice of grating depth, N1 and N2, and grating spacing (between ridges), a resonance with a very narrow spectral width of the order of 0.1 nm is possible.

Variation in the thickness of the coating, which acts as a waveguide at resonance, causes a change in the mode structure of the guide and a corresponding shift in the wavelength required to excite it.

Sensitivity may be increased by making N1 and N2 very close in value and by reducing the thickness of the coating-waveguide. This arises because reducing the thickness of the coating increases the rate of change of propagation velocity therein for a given change in thickness of the coating, and this increases the shift in the critical angle of incidence (for a given wavelength) relative to changes in thickness.

Thus, a suitable sensor for use in accordance with the invention comprises a diffraction grating with N1 similar to N2 and both approximately equal to 1.5, a grating depth of approximately 0.1 microns, grating spacing of approximately 0.3 microns and a coating-waveguide thickness of approximately 0.7 microns, with a similar sensitivity to that of a sensor such as is described in International Patent Publication WO84/02578. When the thickness of the coating-waveguide is reduced below 0.7 microns, this sensitivity can be increased four or fivefold.

Diffraction gratings may be prepared by any conventional means. Thus, for example a grating of plastics may be fabricated by injection moulding from a grating master produced via laser interferometry. A sensor may then be synthesised by depositing a thin layer of a dielectric material onto the grating, for example by the deposition of glass or $SiO_2$ by sputtering or vacuum evaporation respectively.

Alternatively, a glass diffraction grating may be produced by drawing lines in the surface of optically flat glass or by spinning a photoresist onto an optically flat glass substrate, exposing it to an interference pattern, for example as produced by a light interferometer, and then etching the glass surface by exposing the interference pattern in the resist to reactive ions. A glass grating with an ion-impregnated surface may then be constructed. For example, silver and/or lead ions may be bound into the surface layer of the grating by exposing the surface to silver nitrate and/or lead nitrate. This permits migration of silver and/or lead ions into the glass surface and thus establishes a very thin surface region having a refractive index slightly different from the remainder of the glass. The thinness of the surface layer and the similarity of the refractive indices of the substrate and the surface layer are both requirements for giving good sensitivity.

The optical properties of an optical structure coated with a specific binding partner are altered by complex formation between the specific binding partner and the complementary ligand and, in one embodiment of the invention, by comparing the optical characteristics of a standard (untreated) region with those of a treated test region of the surface it is possible to determine qualitatively or quantitatively whether a binding reaction has occurred in the test region. In an alternative embodiment where, for example, the specific binding partner is an antibody, an antibody specific to the antigen to be tested for is adsorbed on or bound to at least one discrete test region on the surface of an optical structure and a protein which is not a specific binding partner for any ligand which may be present in the sample to be tested (denoted herein as a "non-specific protein") is adsorbed on or bound to at least one discrete reference region on said surface. The non-specific protein may be, for example, an inactivated antibody or an antibody raised against a ligand not present in the samples to be tested e.g. where the samples to be tested are human sera, the non-specific protein may be an anti-mouse antibody. Any differences between the non-specific binding of e.g. proteins present in the test sample to either the specific antibody or the non-specific protein can be determined by comparing the optical properties of the test region with the reference region after exposure to a sample which does not contain the antigen to be tested for, so that an appropriate correction can, if necessary, be made. Comparison of the optical characteristics of the test and standard regions of a similar biosensor during or after exposure to a sample to be tested can then provide a measurement of complex formation between the antigen to be tested for and its specific antibody. Each region may comprise a continuous layer of a specific binding partner or each binding partner may be present at discrete intervals within any given region to form a discontinuous layer.

According to the invention the optical structure is irradiated at a fixed angle of incidence with polychromatic light and the reflected and/or transmitted light is analysed to determine any wavelengths of light which are absorbed due to guided wave resonance.

The means for analysing the reflected or transmitted light may be a spectrum detector which determines any wavelengths missing from the reflected or transmitted light, compared to the irradiating light, and which generates a signal indicative of the missing wavelengths. The signal generating means may be calibrated to provide a direct readout.

If a significant wavelength shift is found between the missing part of the spectrum before and after bringing a sample into contact with the coated grating surface, it can be deduced that binding of a complementary material has occurred at the surface and thus that the sample contained a specific ligand known to bind to the coating of its specific binding partner.

A further aspect of the present invention provides apparatus for use in a method as described hereinbefore comprising (A) a sensor having a substrate with a first refractive index N1 and a surface layer with a second refractive index N2, said surface layer being such that, in use, a guided wave mode of resonant radiation may be propagated therein, and said surface layer having adsorbed thereon or bound thereto, either directly or indirectly, a specific binding partner for the ligand it is desired to detect; and (B) a spectrum detector. A source of polychromatic light may also be comprised in said apparatus.

## Claims

1. A method of assaying for a ligand in a sample which comprises
   a) incubating the sample in contact with the surface of an optical structure, said optical structure comprising a substrate with a first refractive index N1 and a surface layer with a second refractive index N2, said surface layer being such that, in use, a guided wave mode of resonant radiation may be propagated therein, and said surface layer having adsorbed thereon or bound thereto, either directly or indirectly, a specific binding partner for the ligand it is desired to detect;
   b) irradiating the optical structure with polychromatic light at a fixed angle to the normal; and
   c) analysing the reflected and/or transmitted light in order to determine whether, and if desired the extent to which and/or rate at which, the absorption of any wavelengths of light as a result of guided wave resonance is altered by formation of a complex between the ligand and the specific binding partner.

2. A method as claimed in claim 1 wherein the optical structure is a diffraction grating.

3. A mehtod as claimed in claim 1 or claim 2 wherein the specific binding partner is an antigen or an antibody.

4. A method as claimed in any one of the preceding claims wherein the refractive indices N1 and N2 are similar (but not precisely equal) and both are approximately 1.5.

5. A method as claimed in any one of the preceding claims wherein the surface layer of refractive index N2 comprises a thin layer of material.

6. A method as claimed in claim 5 wherein the substrate of the optical structure is of glass and the thin layer of material is silica.

7. A method as claimed in claim 5 wherein the substrate of the optical structure is of plastics and the thin layer of material is glass or silica.

8. A method as claimed in any one of claims 1 to 4 wherein the surface layer of refractive index N2 comprises the surface of the substrate impregnated with metal ions.

9. A method as claimed in claim 8 wherein the substrate of the optical structure is of glass and the substrate impregnated with silver and/or lead ions.

10. A method as claimed in any one of the preceding claims wherein the surface layer of refractive index N2 is less than 700 nm thick.

11. Apparatus for use in a method as claimed in claim 1 comprising (A) a sensor having a substrate with a first refractive index N1 and a surface layer with a second refractive index N2, said surface layer being such that, in use, a guided wave mode of resonant radiation may be propagated therein, and said surface layer having adsorbed thereon or bound thereto, either directly or indirectly, a specific binding partner for the ligand it is desired to detect; (B) a spectrum detector; and (C) a source of polychromatic light.

**Patentansprüche**

1. Verfahren zur Bestimmung eines Liganden in einer Probe, welches umfaßt:

   a) Bebrüten der Probe in Kontakt mit der Oberfläche einer optischen Struktur, wobei diese optische Struktur ein Substrat mit einem ersten Brechungsindex N1 und einer Oberflächenschicht mit einem zweiten Brechungsindex N2 umfaßt, wobei diese Oberflächenschicht derart ist, daß bei Gebrauch eine Welle von Resonanzstrahlung darin geführt fortschreiten kann und diese Oberflächenschicht auf ihr adsorbiert oder daran gebunden, enweder direkt oder indirekt, einen spezifischen Bindungspartner für den Liganden, der entdeckt werden soll, hat;

   b) Bestrahlen der optischen Struktur mit polychromatischem Licht bei einem fixierten Winkel gegenüber der Normalen; und

   c) Analysieren des reflektieren und/oder durchgelassenen Lichtes, um festzustellen, ob und gewünschtenfalls in welchem Ausmaß und/oder welcher Geschwindigkeit die Absorption irgendeiner der Wellenlängen des Lichts als Ergebnis der geführten Wellenresonanz geändert ist durch Bildung eines Komplexes zwischen dem Liganden und dem spezifischen Bindungspartner.

2. Verfahren gemäß Anspruch 1, wobei die optische Struktur ein optisches bzw. Beugungsgitter ist.

3. Verfahren gemäß Anspruch 1 oder 2, wobei der spezifische Bindungspartner ein Antigen oder ein Antikörper ist.

4. Verfahren gemäß einem der vorhergehenden Ansprüche, wobei die Beugungs- bzw. Brechungsindices N1 und N2 ähnlich (jedoch nicht genau gleich) sind und beide etwa 1,5 betragen.

5. Verfahren gemäß einem der vorhergehenden Ansprüche, wobei die Oberflächenschicht des Brechungsindex N2 eine dünne Schicht von Material umfaßt.

6. Verfahren gemäß Anspruch 5, wobei das Substrat der optischen Struktur aus Glas ist und die dünne Materialschicht Siliziumdioxid ist.

7. Verfahren gemäß Anspruch 5, wobei das Substrat der optischen Struktur aus Plastik ist und die dünne Materialschicht Glas oder Siliziumdioxid ist.

8. Verfahren gemäß einem der Ansprüche 1 bis 4, wobei die Oberflächenschicht des Brechungsindex N2 die Oberfläche des Substrats imprägniert mit Metallionen umfaßt.

9. Verfahren gemäß Anspruch 8, wobei das Substrat der optischen Struktur aus Glas ist und die Oberflächenschicht die Oberfläche dieses Substrats imprägniert mit Silber- und/oder Bleiionen umfaßt.

10. Verfahren gemäß einem der vorhergehenden Ansprüche, wobei die Oberflächenschicht des Brechungsindex N2 weniger als 700 nm dick ist.

11. Vorrichtung zur Verwendung bei der Methode wie in Anspruch 1 beansprucht, umfassend (A) einen Sensor mit einem Substrat mit einem ersten Brechungsindex N1 und einer Oberflächenschicht mit einem zweiten Brechungsindex N2, wobei diese Oberflächenschicht derart ist, daß beim Gebrauch eine Welle der Resonanzstrahlung darin geführt fartschreiten kann, und diese Oberflächenschicht auf ihr adsorbiert oder an sie gebunden, entweder direkt oder indirekt, einen spezifischen Bindungspartner für den Liganden, der entdeckt werden soll, hat; (B) einen Spektrumdetektor; und (C) eine Quelle für polychromatisches Licht.

**Revendications**

1. Méthode d'essai pour un ligand dans un échantillon, qui comprend:

   a) l'incubation de l'échantillon en contact avec la surface d'une structure optique, cette structure optique comprenant un substrat ayant un premier indice de réfraction N1 et une couche de surface ayant un second indice de réfraction N2, cette couche de surface étant telle qu'en cours d'utilisation, un mode d'onde guidée de radiation résonante peut être propagé dans celle-ci, et sur laquelle couche de surface est adsorbé ou lié, soit directement soit indirectement, un partenaire de liaison spécifique pour le ligand devant être détecté;

b) l'irradiation de la structure optique avec une lumière polychromatique selon un angle déterminé par rapport à la normale; et

c) l'analyse de la lumière réfléchie et/ou transmise pour déterminer si, et si cela est désiré, dans quelle mesure et/ou à quelle vitesse l'absorption de quelconques longueurs d'onde de lumière par suite d'une résonance d'onde guidée, est modifiée par la formation d'un complexe entre le ligand et le partenaire de liaison spécifique.

2. Méthode suivant la revendication 1, dans laquelle la structure optique est un réseau de diffraction.

3. Méthode suivant la revendication 1 ou la revendication 2, dans laquelle le partenaire de liaison spécifique est un antigène ou un anticorps.

4. Méthode suivant l'une quelconque des revendications précédentes, dans laquelle les indices de réfraction N1 et N2 sont similaires (mais pas précisément égaux) et sont tous les deux d'environ 1,5.

5. Méthode suivant l'une quelconque des revendications précédentes, dans laquelle la couche de surface ayant l'indice de réfraction N2 comprend une mince couche de matériau.

6. Méthode suivant la revendication 5, dans laquelle le substrat de la structure optique est en verre et la mince couche de matériau est en silice.

7. Méthode suivant la revendication 5, dans laquelle le substrat de la structure optique est en matière plastique et la mince couche de matériau est en verre ou en silice.

8. Méthode suivant l'une quelconque des revendications 1 à 4, dans laquelle la couche de surface ayant l'indice de réfraction N2 comprend la surface du substrat imprégnée d'ions métalliques.

9. Méthode suivant la revendication 8, dans laquelle le substrat de la structure optique est en verre et la couche de surface comprend la surface de ce substrat imprégnée d'ions argent et/ou plomb.

10. Méthode suivant l'une quelconque des revendications précédentes, dans laquelle la couche de surface ayant l'indice de réfraction N2 a moins de 700 nm d'épaisseur.

11. Appareil utile dans une méthode suivant la revendication 1, comprenant [A] un détecteur ayant un substrat avec un premier indice de réfraction N1 et une couche de surface avec un second indice de réfraction N2, cette couche de surface étant telle qu'en cours d'utilisation, un mode d'onde guidée de radiation résonante peut être propagé dans celle-ci, et sur laquelle couche de surface est adsorbé ou lié, soit directement soit indirectement, un partenaire de liaison spécifique pour le ligand devant être détecté; [B] un détecteur de spectre; et [C] une source de lumière polychromatique.